# EUROPEAN PATENT SPECIFICATION

(11) **EP 0 811 683 B1**
(45) Date of publication and mention of the grant of the patent: **23.01.2008**
(21) Application number: 96108888.7
(22) Date of filing: 03.06.1996
(51) Int. Cl.: C12N 15/12, C07K 16/26, C12N 5/20, G01N 33/577, G01N 33/574

(54) **Antibodies to human gastrin-releasing peptide precursor and use thereof**
Antikörper gegen den menschlichen gastrinfreisetzenden Peptidvorläufer und deren Verwendung
Anticorps contre le précurseur humain de la peptide libérant la gastrine et son utilisation

(43) Date of publication of application: 10.12.1997
(73) Proprietor: Yamaguchi, Ken, Chuo-ku, Tokyo (JP); Advanced Life Science Institute, Inc., Wako-shi Saitama (JP)
(72) Inventor: Yamaguchi, Ken, 1-1, Tsukiji 5-chome, Chuo-ku, Tokyo (JP); Miyake, Yoshio, 1-1, Tsukiji 5-chome, Chuo-ku, Tokyo (JP)
(74) Representative: Klunker . Schmitt-Nilson . Hirsch

(56) References cited:
- WO-A-94/02018
- CA-A- 2 089 212
- CLINICAL CHEMISTRY, vol. 41, no. 4, April 1995, WASHINGTON, DC, USA, pages 537-543, XP002017660 K. AOYAGI ET AL.: "Enzyme immunoassay of immunoreactive progastrin-releasing peptide(31-98) as tumor marker for small-cell lung carcinoma: development and evaluation."
- RESPIRATION, vol. 63, no. 2, 1996, BASEL, SWITZERLAND, pages 106-110, XP000608937 N. SHIJUBO ET AL.: "Elevated progastrin-releasing peptide (31-98) concentrations in pleural effusions due to small-cell lung carcinoma."
- PROCEEDINGS OF THE NATIONAL ACADEMY OF SCIENCES OF THE USA, vol. 81, no. 18, - September 1984 WASHINGTON, DC, USA, pages 5699-5703, XP002017661 E. SPINDEL ET AL.: "Cloning and characterization of cDNAs encoding human gastrin-releasing peptide."
- THE JOURNAL OF BIOLOGICAL CHEMISTRY, vol. 263, no. 4, 5 February 1988, BALTIMORE, MD, USA, pages 1928-1932, XP002017662 J. REEVE ET AL.: "Multiple gastrin-releasing peptide gene-associated peptides are produced by a human small cell lung cancer line."

### SEQUENCE LISTING

(1) GENERAL INFORMATION:
   (i) APPLICANTS:
   (ii) TITLE OF INVENTION:
      Antibodies to Human Gastrin-Releasing Peptide Precursor and Use Thereof
   (iv) CORRESPONDENCE ADDRESS:
   (v) COMPUTER READABLE FORM:
      (A) MEDIUM TYPE:
      (B) COMPUTER:
      (C) OPERATING SYSTEM:
      (D) SOFTWARE:
   (vi) CURRENT APPLICATION DATE:
   (vii) PRIOR APPLICATION DATE:
   (viii) ATTORNEY/AGENCY INFORMATION:
   (ix) TELECOMMUNICATION INFORMATION:
(2) INFORMATION FOR SEQ ID NO: 1
   (i) SEQUENCE CHARACTERISTICS:
      (A) LENGTH: 204 base pairs
      (B) TYPE: Nucleic acid
      (C) STRANDEDNESS: Double
      (D) TOPOLOGY: Linear
   (ii) MOLECULE TYPE: Synthetic DNA
   (xi) SEQUENCE DESCRIPTION: SEQ ID No: 1
   AGT ACT GGT GAG AGC TCT TCT GTT TCT GAA CGT GGA TCC CTT AAG CAG 48
   Ser Thr Gly Glu Ser Ser Ser Val Ser Glu Arg Gly Ser Leu Lys Gln
                     5                  10                  15
   CAG CTT CGC GAA TAC ATC CGT TGG GAA GAA GCT GCT CGT AAC CTG CTA 96
   Gln Leu Arg Glu Tyr Ile Arg Trp Glu Glu Ala Ala Arg Asn Leu Leu
                20                  25                  30
   GGC CTG ATC GAA GCT AAA GAA AAC CGT AAC CAC CAG CCG CCG CAG CCG 144
   Gly Leu Ile Glu Ala Lys Glu Asn Arg Asn His Gln Pro Pro Gln Pro
            35                  40                  45
   AAA GCT TTA GGT AAC CAG CAG CCG TCT TGG GAC TCT GAA GAC TCT TCG 192
   Lys Ala Leu Gly Asn Gln Gln Pro Ser Trp Asp Ser Glu Asp Ser Ser
        50                  55                  60
   AAC TTT AAA GAC                                                 204
   Asn Phe Lys Asp
    65
(2) INFORMATION FOR SEQ ID NO: 2
   (i) SEQUENCE CHARACTERISTICS:
      (A) LENGTH: 47 bases
      (B) TYPE: Nucleic acid
      (C) STRANDEDNESS: Single
      (D) TOPOLOGY: Linear
   (ii) MOLECULE TYPE: Synthetic DNA
   (xi) SEQUENCE DESCRIPTION: SEQ ID NO: 2
      AATTCATGAG TACTGGTGAG AGCTCTTCTG TTTCTGAACG TGGATCC 47
(2) INFORMATION FOR SEQ ID NO: 3
   (i) SEQUENCE CHARACTERISTICS:
      (A) LENGTH: 54 bases
      (B) TYPE: Nucleic acid
      (C) STRANDEDNESS: Single
      (D) TOPOLOGY: Linear
   (ii) MOLECULE TYPE: Synthetic DNA
   (xi) SEQUENCE DESCRIPTION: SEQ ID NO: 3
   CTTAAGCAGC AGCTTCGCGA ATACATCCGT TGGGAAGAAG CTGCTCGTAA 50
   CCTG 54
(2) INFORMATION FOR SEQ ID NO: 4
   (i) SEQUENCE CHARACTERISTICS:
      (A) LENGTH: 54 bases
      (B) TYPE: Nucleic acid
      (C) STRANDEDNESS: Single
      (D) TOPOLOGY: Linear
   (ii) MOLECULE TYPE: Synthetic DNA
   (xi) SEQUENCE DESCRIPTION: SEQ ID NO: 4
   CTAGGCCTGA TCGAAGCTAA AGAAAACCGT AACCACCAGC CGCCGCAGCC 50
   GAAA 54
(2) INFORMATION FOR SEQ ID NO: 5
   (i) SEQUENCE CHARACTERISTICS:
      (A) LENGTH: 64 bases
      (B) TYPE: Nucleic acid
      (C) STRANDEDNESS: Single
      (D) TOPOLOGY: Linear
   (ii) MOLECULE TYPE: Synthetic DNA
   (xi) SEQUENCE DESCRIPTION: SEQ ID NO: 5
   GCTTTAGGTA ACCAGCAGCC GTCTTGGGAC TCTGAAGACT CTTCGAACTT 50
   TAAAGACTAA TAAG 64
(2) INFORMATION FOR SEQ ID NO: 6
   (i) SEQUENCE CHARACTERISTICS:
      (A) LENGTH: 49 bases
      (B) TYPE: Nucleic acid
      (C) STRANDEDNESS: Single
      (D) TOPOLOGY: Linear
   (ii) MOLECULE TYPE: Synthetic DNA
   (xi) SEQUENCE DESCRIPTION: SEQ ID NO: 6
      CTTAAGGGAT CCACGTTCAG AAACAGAAGA GCTCTCACCA GTACTCATG 49
(2) INFORMATION FOR SEQ ID NO: 7
   (i) SEQUENCE CHARACTERISTICS:
      (A) LENGTH: 54 bases
      (B) TYPE: Nucleic acid
      (C) STRANDEDNESS: Single
      (D) TOPOLOGY: Linear
   (ii) MOLECULE TYPE: Synthetic DNA
   (xi) SEQUENCE DESCRIPTION: SEQ ID NO: 7
   GCCTAGCAGG TTACGAGCAG CTTCTTCCCA ACGGATGTAT TCGCGAAGCT 50
   GCTG 54
(2) INFORMATION FOR SEQ ID NO: 8
   (i) SEQUENCE CHARACTERISTICS:
      (A) LENGTH: 54 bases
      (B) TYPE: Nucleic acid
      (C) STRANDEDNESS: Single
      (D) TOPOLOGY: Linear
   (ii) MOLECULE TYPE: Synthetic DNA
   (xi) SEQUENCE DESCRIPTION: SEQ ID NO: 8
      TAAAGCTTTC GGCTGCGGCG GCTGGTGGTT ACGGTTTTCT TTAGCTTCGA TCAG 54
(2) INFORMATION FOR SEQ ID NO: 9
   (i) SEQUENCE CHARACTERISTICS:
      (A) LENGTH: 62 bases
      (B) TYPE: Nucleic acid
      (C) STRANDEDNESS: Single
      (D) TOPOLOGY: Linear
   (ii) MOLECULE TYPE: Synthetic DNA
   (xi) SEQUENCE DESCRIPTION: SEQ ID NO: 9
   TCGACTTATT AGTCTTTAAA GTTCGAAGAG TCTTCAGAGT CCCAAGACGG 50
   CTGCTGGTTA CC 62
(2) INFORMATION FOR SEQ ID NO: 10
   (i) SEQUENCE CHARACTERISTICS:
      (A) LENGTH: 22
      (B) TYPE: Amino acid
      (D) TOPOLOGY: Linear
   (ii) MOLECULE TYPE: Synthetic peptide
   (xi) SEQUENCE DESCRIPTION: SEQ ID NO: 10
   Ser Thr Gly Glu Ser Ser Ser Val Ser Glu Arg Gly Ser Leu Lys Gln
                    5                  10                  15
   Gln Leu Arg Glu Tyr Ile
                20
(2) INFORMATION FOR SEQ ID NO: 11
   (i) SEQUENCE CHARACTERISTICS:
      (A) LENGTH: 19
      (B) TYPE: Amino acid
      (D) TOPOLOGY: Linear
   (ii) MOLECULE TYPE: Synthetic peptide
   (xi) SEQUENCE DESCRIPTION: SEQ ID NO: 11
   Leu Lys Gln Gln Leu Arg Glu Tyr Ile Arg Trp Glu Glu Ala Ala Arg
                    5                  10                  15
   Asn Leu Leu
(2) INFORMATION FOR SEQ ID NO: 12
   (i) SEQUENCE CHARACTERISTICS:
      (A) LENGTH: 21
      (B) TYPE: Amino acid
      (D) TOPOLOGY: Linear
   (ii) MOLECULE TYPE: Synthetic peptide
   (xi) SEQUENCE DESCRIPTION: SEQ ID NO: 12
   Asn Arg Asn His Gln Pro Pro Gln Pro Lys Ala Leu Gly Asn Gln Gln
                    5                  10                  15
   Pro Ser Trp Asp Ser
                20

### SEQUENCE LISTING

(1) GENERAL INFORMATION:
   (i) APPLICANT:
      (A) NAME: Ken Yamaguchi
      (B) STREET: 1-1, Tsukiji 5-chome Chou-ku
      (C) CITY: Tokyo
      (E) COUNTRY: Japan
      (F) POSTAL CODE (ZIP): none

      (A) NAME: Tonen Corporation
      (B) STREET: 1-1, Hitotsubashi 1-chome Chiyoda-ku
      (C) CITY: Tokyo
      (E) COUNTRY: Japan
      (F) POSTAL CODE (ZIP): none

      (A) NAME: Terumo Corporation
      (B) STREET: 44-1, Hatagaya 2-chome Shibuya-ku
      (C) CITY: Tokyo
      (E) COUNTRY: Japan
      (F) POSTAL CODE (ZIP): none
   (ii) TITLE OF INVENTION: Antibodies to Human Gastrin-Releasing Peptide Precursor and Use Thereof
   (iii) NUMBER OF SEQUENCES: 13
   (iv) COMPUTER READABLE FORM:
      (A) MEDIUM TYPE: Floppy disk
      (B) COMPUTER: IBM PC compatible
      (C) OPERATING SYSTEM: PC-DOS/MS-DOS
      (D) SOFTWARE: PatentIn Release #1.0, Version #1.30 (EPO)
   (v) CURRENT APPLICATION DATA:
      APPLICATION NUMBER: EP 96108888.7
(2) INFORMATION FOR SEQ ID NO: 1:
   (i) SEQUENCE CHARACTERISTICS:
      (A) LENGTH: 204 base pairs
      (B) TYPE: nucleic acid
      (C) STRANDEDNESS: double
      (D) TOPOLOGY: linear
   (ii) MOLECULE TYPE: other nucleic acid
      (A) DESCRIPTION: /desc = "synthetic DNA"
   (ix) FEATURE:
      (A) NAME/KEY: CDS
      (B) LOCATION:1..204
   (xi) SEQUENCE DESCRIPTION: SEQ ID NO: 1:
   AGT ACT GGT GAG AGC TCT TCT GTT TCT GAA CGT GGA TCC CTT AAG CAG      48
   Ser Thr Gly Glu Ser Ser Ser Val Ser Glu Arg Gly Ser Leu Lys Gln
    1               5                  10                  15
   CAG CTT CGC GAA TAC ATC CGT TGG GAA GAA GCT GCT CGT AAC CTG CTA      96
   Gln Leu Arg Glu Tyr Ile Arg Trp Glu Glu Ala Ala Arg Asn Leu Leu
               20                  25                  30
   GGC CTG ATC GAA GCT AAA GAA AAC CGT AAC CAC CAG CCG CCG CAG CCG     144
   Gly Leu Ile Glu Ala Lys Glu Asn Arg Asn His Gln Pro Pro Gln Pro
           35                  40                  45
   AAA GCT TTA GGT AAC CAG CAG CCG TCT TGG GAC TCT GAA GAC TCT TCG     192
   Lys Ala Leu Gly Asn Gln Gln Pro Ser Trp Asp Ser Glu Asp Ser Ser
       50                  55                  60
   AAC TTT AAA GAC                                                     204
   Asn Phe Lys Asp
    65
(2) INFORMATION FOR SEQ ID NO: 2:
   (i) SEQUENCE CHARACTERISTICS:
      (A) LENGTH: 68 amino acids
      (B) TYPE: amino acid
      (D) TOPOLOGY: linear
   (ii) MOLECULE TYPE: protein
   (xi) SEQUENCE DESCRIPTION: SEQ ID NO: 2:
   Ser Thr Gly Glu Ser Ser Ser Val Ser Glu Arg Gly Ser Leu Lys Gln
    1               5                  10                  15
   Gln Leu Arg Glu Tyr Ile Arg Trp Glu Glu Ala Ala Arg Asn Leu Leu
               20                  25                  30
   Gly Leu Ile Glu Ala Lys Glu Asn Arg Asn His Gln Pro Pro Gln Pro
           35                  40                  45
   Lys Ala Leu Gly Asn Gln Gln Pro Ser Trp Asp Ser Glu Asp Ser Ser
       50                  55                  60
   Asn Phe Lys Asp
    65
(2) INFORMATION FOR SEQ ID NO: 3:
   (i) SEQUENCE CHARACTERISTICS:
      (A) LENGTH: 47 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "synthetic DNA"
   (xi) SEQUENCE DESCRIPTION: SEQ ID NO: 3:
      AATTCATGAG TACTGGTGAG AGCTCTTCTG TTTCTGAACG TGGATCC 47
(2) INFORMATION FOR SEQ ID NO: 4:
   (i) SEQUENCE CHARACTERISTICS:
      (A) LENGTH: 54 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "synthetic DNA"
   (xi) SEQUENCE DESCRIPTION: SEQ ID NO: 4:
      CTTAAGCAGC AGCTTCGCGA ATACATCCGT TGGGAAGAAG CTGCTCGTAA CCTG 54
(2) INFORMATION FOR SEQ ID NO: 5:
   (i) SEQUENCE CHARACTERISTICS:
      (A) LENGTH: 54 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "synthetic DNA"
   (xi) SEQUENCE DESCRIPTION: SEQ ID NO: 5:
      CTAGGCCTGA TCGAAGCTAA AGAAAACCGT AACCACCAGC CGCCGCAGCC GAAA 54
(2) INFORMATION FOR SEQ ID NO: 6:
   (i) SEQUENCE CHARACTERISTICS:
      (A) LENGTH: 64 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "synthetic DNA"
   (xi) SEQUENCE DESCRIPTION: SEQ ID NO: 6:
   GCTTTAGGTA ACCAGCAGCC CTCTTGGGAC TCTGAAGACT CTTCGAACTT TAAAGACTAA 60
   TAAG 64
(2) INFORMATION FOR SEQ ID NO: 7:
   (i) SEQUENCE CHARACTERISTICS:
      (A) LENGTH: 49 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "synthetic DNA"
   (xi) SEQUENCE DESCRIPTION: SEQ ID NO: 7:
      CTTAAGGGAT CCACGTTCAG AAACAGAAGA GCTCTCACCA GTACTCATG 49
(2) INFORMATION FOR SEQ ID NO: 8:
   (i) SEQUENCE CHARACTERISTICS:
      (A) LENGTH: 54 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "synthetic DNA"
   (xi) SEQUENCE DESCRIPTION: SEQ ID NO: 8:
      GCCTAGCAGG TTACGAGCAG CTTCTTCCCA ACGGATGTAT TCGCGAAGCT GCTG 54
(2) INFORMATION FOR SEQ ID NO: 9:
   (i) SEQUENCE CHARACTERISTICS:
      (A) LENGTH: 54 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "synthetic DNA"
   (xi) SEQUENCE DESCRIPTION: SEQ ID NO: 9:
      TAAAGCTTTC GGCTGCGGCG GCTGGTGGTT ACGGTTTTCT TTAGCTTCGA TCAG 54
(2) INFORMATION FOR SEQ ID NO: 10:
   (i) SEQUENCE CHARACTERISTICS:
      (A) LENGTH: 62 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "synthetic DNA"
   (xi) SEQUENCE DESCRIPTION: SEQ ID NO: 10:
   TCGACTTATT AGTCTTTAAA GTTCGAAGAG TCTTCAGAGT CCCAAGACGG CTGCTGGTTA      60
   CC                                                                     62
(2) INFORMATION FOR SEQ ID NO: 11:
   (i) SEQUENCE CHARACTERISTICS:
      (A) LENGTH: 22 amino acids
      (B) TYPE: amino acid
      (C) STRANDEDNESS:
      (D) TOPOLOGY: linear
   (ii) MOLECULE TYPE: peptide
   (xi) SEQUENCE DESCRIPTION: SEQ ID NO: 11:
   Ser Thr Gly Glu Ser Ser Ser Val Ser Glu Arg Gly Ser Leu Lys Gln
   1               5                   10                  15
   Gln Leu Arg Glu Tyr Ile
               20
(2) INFORMATION FOR SEQ ID NO: 12:
   (i) SEQUENCE CHARACTERISTICS:
      (A) LENGTH: 19 amino acids
      (B) TYPE: amino acid
      (C) STRANDEDNESS:
      (D) TOPOLOGY: linear
   (ii) MOLECULE TYPE: peptide
   (xi) SEQUENCE DESCRIPTION: SEQ ID NO: 12:
   Leu Lys Gln Gln Leu Arg Glu Tyr Ile Arg Trp Glu Glu Ala Ala Arg
   1               5                   10                  15
   Asn Leu Leu
(2) INFORMATION FOR SEQ ID NO: 13:
   (i) SEQUENCE CHARACTERISTICS:
      (A) LENGTH: 21 amino acids
      (B) TYPE: amino acid
      (C) STRANDEDNESS:
      (D) TOPOLOGY: linear
   (ii) MOLECULE TYPE: peptide
   (xi) SEQUENCE DESCRIPTION: SEQ ID NO: 13:
   Asn Arg Asn His Gln Pro Pro Gln Pro Lys Ala Leu Gly Asn Gln Gln
   1 5 10 15
   Pro Ser Trp Asp Ser
   20

## Description

### BACKGROUND OF THE INVENTION

### 1. Field of the Invention

The present invention relates to antibodies to human gastrin-releasing peptide (GRP) precursor and the use of the antibodies as a diagnostic agent for small cell lung cancer.

### 2. Related Art

It is known that cancer cells produce substances specific to the cancer cells as well as substances common to normal cells and cancer cells. It has become possible to diagnose the characteristics of cancer cells and patients with cancer by measuring the cancer-specific substances. Substances specifically produced by cancer cells include oncogene products and growth factors, which are responsible for oncogenesis, growth and developments of cells. Moreover, it is considered that the production of carcinoembryotic proteins, hormones and enzymes and the like are characteristics of the oncogenesis. Therefore, if one of substances which define cancer cells, i.e., so-called tumor markers, can be assayed with high sensitivity, diagnosis of cancers becomes possible.

Since the presence of neuroendocrine particles in small cell lung cancer was observed in 1968, it has been asserted that small cell lung cancer is derived from neuroendocrine cells, and at present, is included in APUD (amine precursor uptake and decarboxylation)-type tumors, and is distinguished from other non-small cell lung cancers which are epithelial tumors. It has been known that small cell lung cancer shows the characteristics of the neuroendocrine cells in a cytobiological study, and is reported to produce peptide hormones such as serotonin, adrenocorticotropic hormone (ACTH), calcitonin, gastrin releasing peptide (GRP) etc., to exhibit high L-dopa decarboxylase (L-DDC) activity characteristic to APUD-type cells or tumors, and to exhibit high activities of neuron specific enolase (NSE) and creatine kinase BB (CK-BB), which are specific to neuron cells.

Analysis of a surface antigen of small cell lung cancer based on various biological properties of the cells as indicators using a monoclonal antibody was started with a report by Minna, Science 214, 1246-1248, 1981, and has been well developed by many researchers. Assay systems so far practically used as tumor markers include those using carcinoembryonic antigens such as carcinoembryonic antigen (CEA), α-fetoprotein (AFP), Carbohydrate Antigen 125 (CA125); enzymes such as NSE, L-DDC, CK-BB; hormone-related substances such as ACHT, alcohol dehydrogenase (ADH). However, the positive ratio for sera of patients with cancer obtained using the above assay systems is at most 50 to 60%, while frequently patients having cancer provide negative results.

So far, GRP is known as one of tumor markers of small cell lung cancer. GRP is a peptide consisting of 27 amino acids extracted from the stomach of swine by McDonald in 1978, and has activities to stimulate secretion of gastric acid, various hormones etc. There are three precursor proteins different in their C-terminal structure due to alternative splicing of RNA. Recently, the production of GRP as an autocrine growth factor in small cell lung cancer was found, and it is interesting as a tumor marker.

In patients with small cell lung cancer, about 80% of the cases provide increased blood GRP concentration. Moreover, even in the cases in an early phase, the blood GRP concentration is at an increased level, and therefore diagnosis of cancer using GRP as a marker promises to be highly effective. However, conventional assay methods for GRP use antibodies to an active peptide, GRP(1-27), and its sensitivity is too low to be practically used. It is supposed that one of main reasons for the difficulty in measuring a serum GRP concentration using an antibody to GRP(1-27) is the instability of GRP(1-27) in the blood.

Holst et al. (J. Clin. Oncol. 7, 1831-1838, (1989)) developed a radioimmunoassay (RIA) system using polyclonal antibodies to a synthetic peptide corresponding to the portion from the 42 to 53 positions of the C-terminal flanking peptide of GRP precursor, and demonstrated that GRP precursor protein can be a powerful diagnostic marker for small cell lung cancer. However this system was not practical because it provided an insufficient positive ratio due to its sensitivity. Because the antigen protein used was a part of GRP precursor, the resulting antibody had low sensitivity and specificity antibody. Moreover, the low sensitivity of the assay system needed an extraction of the analyte protein from a large amount of a sample, resulting in difficulty in clinical application of the system. GRP precursor protein present in the blood is a macromolecule having a molecular weight of 8,000 to 13,000. Therefore, in an assay system using antibodies to GRP(43-52) which is a part of GRP precursor, sensitivity and specificity are limited.

In normal cells, a protein is produced in the rough-surfaced endoplasmic reticulum, concentrated in the Golgi apparatus resulting in formation of secretory granules in which the protein is packed, and extracellularly secreted through the so-called regulated pathway. Since the secretory granules contain proteolytic enzymes, a precursor of the protein can be adequately processed when passing through the regulated pathway. On the other hand, in cancer cells, since the rough-surfaced endoplasmic reticulum is remarkably developed while the number of the secretory granules is small, then when GRP is produced and secreted, GRP precursor is extracellularly secreted through the constitutive pathway from the rough-surfaced endoplasmic reticulum without being affected by action of any proteolytic enzymes, rather than passing through the regulated pathway involving the secretory particles. Therefore, the blood of patients with cancer contains precursor GRP and flanking peptides in addition to an active peptide GRP(1-27).

Compared with GRP(1-27), active site-free flanking peptide of GRP precursor is expected to be stably present at a high concentration in the blood.

### SUMMARY OF THE INVENTION

Accordingly, the present invention provides a new method to detect fragment of GRP precursor by using monoclonal antibodies to GRP precursor (70-90) both as a first and second antibody. This method is in particular suitable for diagnosis of small cell lung cancer.

### BRIEF EXPLANATION OF THE DRAWINGS

Figure 1 represents a nucleotide sequence of DNA coding for GRP(31-98) and of oligonucleotides for synthesizing the DNA.
Fig. 2 represents a process for the construction of an expression plasmid pAT-TrpE-GRP(31-98) for the production of GRP(31-98).
Fig. 3 is a graph showing that GRP(31-98) is more stable than GRP(1-27) in serum or plasma.
Fig. 4 is a graph showing affinity to proGRP of various monoclonal antibodies of the present invention.
Fig. 5 is a graph showing the relationship between the concentration of GRP(31-98) and an amount of polyclonal antibody captured on a solid phase when the polypeptide GRP(31-98) immobilized on the solid phase and different concentrations of the peptide GRP(31-52) competitively bind to the anti-GRP(31-98) polyclonal antibody.
Fig. 6 is the same graph as Fig. 5, except that the immobilized peptide is GRP(31-52).
Fig. 7 is the same graph as Fig. 5, except that the immobilized peptide is GRP(44-62).
Fig. 8 is the same graph as Fig. 5, except that the immobilized peptide is GPR(70-90).
Fig. 9 shows a standard curve for assaying GRP(31-98) by ELISA when a mixture of monoclonal antibodies 2B10 and 3G2 is immobilized on a solid phase and a peroxidase-labeled anti-GRP(31-98) polyclonal antibody is used as a second antibody.
Fig. 10 represents an elution pattern of peptides in a serum or urine sample by gel filtration chromatography, assayed using two monoclonal antibodies specific to GRP(70-90).

### PREFERRED EMBODIMENT FOR CARRYING OUT THE INVENTION

### (1) Antigen protein and process for preparation thereof

In a process for in vivo production of GRP, three GRP mRNAs are generated by alternative splicing of a mRNA, and three precursor proteins having a common amino acid sequence up to the 98 position including the active site but having different C-terminal structures are formed. The GRP precursor antigen used in the present invention is a peptide having an amino acid sequence from the 31th Ser to 98th Asp of the precursor protein, which is common between the three GRP precursors and does not contain a GRP active region. Note that the number of amino acids used in the present invention is determined by taking the N-terminal amino acid Val of mature GRP as the first position. Therefore, the mature GRP is represented by GRP(1-27), and the present antigen peptide is represented by GRP(31-98).

An amino acid sequence of the present GRP precursor antigen GRP(31-98) and an example of nucleotide sequences coding therefor are shown in SEQ ID NO: 1.

The antigen peptide of the present invention can be prepared by chemical synthesis or genetic engineering. Since chemical synthesis of peptides having more than 40 amino acids is difficult, genetic engineering is preferable.

The production of the present antigen peptide by genetic engineering can be carried out by transforming a host such as E*.* coli with an expression vector capable of expressing the present antigen peptide in the host such as E. coli, culturing the transformant, and recovering the antigen peptide from the cultured bacterial cells.

Although the above-mentioned expression vector may contain any nucleotide sequence coding for the present antigen polypeptide, in the case of the E. coli host, a nucleotide sequence comprising codons frequently used in E. coli and not containing palindrome is preferable.

As a nucleotide sequence coding for the amino acid sequence of GRP(31-98), the nucleotide sequence shown in SEQ ID NO: 1 is preferable.

A promoter such as tryptophan promoter is present upstream of the coding region for the present peptide to enhance transcription efficiency in a host used such as E. coli. Although the peptide coding region may be present immediately downstream of the promoter, the coding region may be present downstream of a coding region for a protein inherently produced by a host such as E. coli Trp E protein, as described hereinafter. In the latter case, the present peptide may be obtained as a fusion protein.

An expression vector of the present invention, similar to conventional expression vectors, contains a selective marker such as antibiotic resistance and an origin of replication for replicating in a host such as E. coli. Moreover, a translational stop codon is positioned downstream of the peptide coding region. As a starting material for the construction of the expression vector, for example, pUC9, pBR322, and other vectors such as commercially available vectors may be used.

The above-mentioned expression vector may be constructed by synthesizing the present peptide coding region by a known synthetic process such as the phosphoramidite method, and cloning it into a vector such as a known vector expressed in a host such as E. coli host. In Examples described hereinafter, the GRP coding region was inserted into TrpE gene or TGF-α gene (see Japanese Patent Application No. 63-28908) present downstream of E. coli tryptophan promoter.

Transformation with an expression vector can be carried out according to a conventional procedure. Culturing conditions of a host such as E. coli are also conventional.

The present peptide produced by a host such as E. coli transformed with a vector can be isolated and purified by, for example, collecting cells by, for example, centrifugation, disrupting the cells by a well known lysozyme treatment and/or ultrasonication, and applying to the disruptant gel filtration chromatography or the like. Definite conditions for isolation and purification of the peptide are described hereinafter in Examples.

### (2) Production of antibody

A mouse such as a Balb/C mouse is periodically immunized by peritoneal or subcutaneous administration of the present antigen alone or as an antigen prepared by binding the present antigen with a hapten such as bovine serum albumin (BSA), keyhole limpet hemocyanin (KLH) etc., in a mixture with Freund's complete adjuvant. After antibody titer in the blood is increased, the mouse is boosted by administrating the present antibody through a tail vein of the mouse. After that, the spleen is removed and spleen cells are fused with appropriate myeloid cells such as mouse myeloid cells. This procedure can be carried out according to Kohler and Milstein, Nature 256, p495-497, 1975, to obtain hybridoma.

The hybridoma thus obtained are cultured in an appropriate medium, and a hybridoma cell line producing an antibody specifically reactive with the present antigen is selected and cloned. Next, the produced monoclonal antibodies are recovered by, for example, column chromatography.

Polyclonal antibodies can be prepared by periodically immunizing an animal such as a guinea-pig, rabbit, goat, sheep, etc., with the present antigen alone, or in the form of conjugate with bovine serum albumin (BSA) or keyhole limpet hemocyanin (KLH) etc. by means of a foot pad, intramuscular injection or subcutaneous injection, as a mixture with Freund's complete adjuvant. An increase of antibody titer in the blood is tested, the whole blood is obtained and polyclonal antibodies are recovered by column chromatography or the like.

The monoclonal antibodies or polyclonal antibodies thus obtained can be used to carry out ELISA or other immunoassays such as IRMA, RIA, FIA, CIA, etc. to measure GRP precursor for example in a blood, urine, and biological fluids sample.

### (3) Characterization and use of the monoclonal antibodies

The present monoclonal antibodies such as those produced by the present hybridoma cell lines such as proGRP-1E2, proGRP-2B10, proGRP-20D2, proGRP-3H1, proGRP-3D6, proGRP-3G2 and proGRP-4C9, react with the peptide GRP(31-98) and other fragments of precursor of human gastrin-releasing peptide, and therefore, can be used for detection of fragments of the precursor of human gastrin-releasing peptide, which is an indicative of lung cancer, especially small cell lung cancer.

According to further characterization, monoclonal antibodies produced by hybridomas 2B10, 1E2, 20D2, 3G2 and 3H1 specifically react with GRP(70-90), while monoclonal antibody produced by hybridoma 3D6 reacts with GRP(44-62), and monoclonal antibody produced by hybridoma 4C9 reacts with GRP(31-52).

On the other hand, from analysis of peptides in a serum sample and urine sample, it was found that the serum sample contains peptides having a molecular weight of 10,000 to 12,000, while the urine sample contains shorter peptides having a molecular weight of 2,500 to 7,000, comprising mainly GRP(70-90). Therefore it is considered that the precursor of human gastrin-releasing peptide is decarded in the blood during the circulation stably maintaining a short fragment GRP(70-90), and the fragment is excreted into the urine.

In addition, it was confirmed that fragments of precursor of human gastrin releasing peptide in urine sample can be detected and assayed by using an immuno assay such as ELISA using monoclonal antibodies reactive with fragments of precursor of human gastrin-releasing peptide by using both for a first antibody and a second antibody, while the fragment in urine cannot be detected by an immunoassay using a combination of a monoclonal antibody and a polyclonal antibody, to the fragments of precursor of human gastrin-releasing peptide.

Accordingly, the present invention provides a method for diagnosis of small cell lung cancer by detecting a fragment of precursor of human gastrin-releasing peptide by an immunoassay characterized by using monoclonal antibodies to the precursor of human gastrin-releasing peptide both as a first antibody and a second antibody.

The immunoassay may be ELISA, EIA, FIA, RIA aggutiration method, immuno chromatoassay, and preferably ELISA. In the immunoassay, a monoclonal antibody as a first antibody and a monoclonal antibody as a second antibody can be used. In addition as the first antibody or the second antibody, a mixture of more than one different monoclonal antibody can be used.

### EXAMPLES

The present invention will now be further illustrated by, but is by no means limited to, the following examples.

### Example 1. Preparation of GRP(31-98)

### (1) Construction of cloning vector

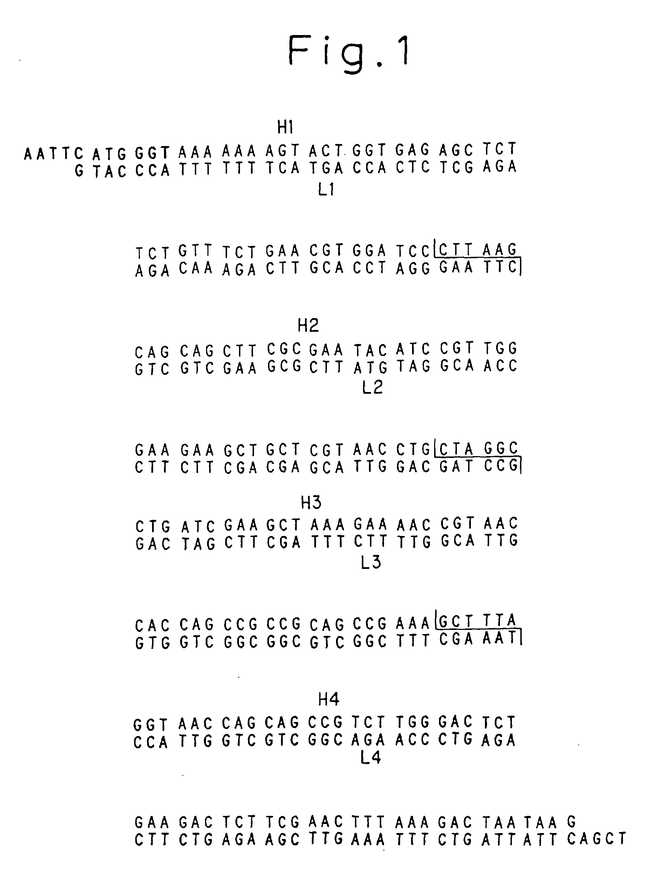

As shown in Fig. 1, GRP gene is divided to DNA fragments of about 60 bases, and each DNA fragment was synthesized by phosphoramidite method.

**Table 1**

| Fragment | SEQ ID NO | Fragment | SEQ ID NO |
|---|---|---|---|
| H1 | 2 | L1 | 6 |
| H2 | 3 | L2 | 7 |
| H3 | 4 | L3 | 8 |
| H4 | 5 | L4 | 9 |

The synthesized DNA fragments were purified by reversed phase chromatography and ligated by T4 DNA ligase to obtain the GRP gene.

The gene thus obtained having a restriction enzyme EcoRI site and SalI site at the 5'- and 3'- termini respectively was inserted into an EcoRI/SalI-digested cloning vector pUC9. The ligation product was used to transform E.coli JM107, which was then cultured overnight in L medium in the presence of 40 µg/ml ampicillin, isopropyl thiogalactoside (IPTG) and X-gal to obtain a candidate strain.

Plasmid was extracted from the candidate strain, and the nucleotide sequence of the inserted gene was tested by the Sanger method to confirm that the inserted gene had the expected nucleotide sequence. E. coli containing a cloning vector comprising a desired GRP gene was designated as pUC-GRP(31-98)/JM107, and the cloning vector was designated as pUC-GRP(31-98).

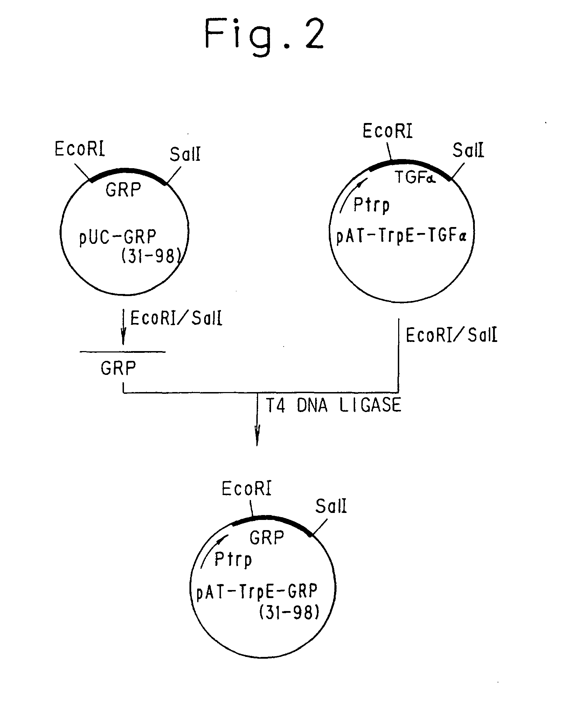

### (2) Construction of expression vector (Fig. 2)

The vector pUC-GRP(31-98) was digested with EcoRI and SalI, and a GRP gene fragment of about 220 base pairs was extracted. On the other hand, an expression vector pAT-TrpE-TGF-α was digested with restriction enzymes EcoRI and SalI to obtain a larger fragment, which was then ligated with the GPR gene fragment using T4 DNA ligase. The ligation product was used to transform E.coli HB101, which was then cultured overnight in L medium in the presence of 40 µg/ml ampicillin to obtain a candidate strain. The candidate strain was designated as pAT-TrpE-GRP(31-98)/HB101, and the expression vector thus obtained was designated as pAT-TrpE-GRP(31-98).

### (3) Purification of expressed polypeptide

The above-constructed strain containing the expression vector, pAT-TrpE-GRP(31-98)/HB101 was cultured and the expression of recombinant protein was tested. Namely, the strain pAT-TrpE-GRP(31-98)/HB101 was cultured overnight in 32 ml of LB medium containing 40 µg/ml ampicillin, and the resulting culture was inoculated to 3.2L of M9 medium containing 0.5% casamino acid and culturing was carried out at 37°C. When the absorbance at 600 nm reached 0.4, indolacrylic acid (IAA) was added to the culture to a final concentration of 30 µg/ml, and the culturing was further continued for 20 hours. The culture was centrifuged to collect 10g of cells. The collected cells were suspended in a 100 mM Tris-HCl (pH 8.0) buffer containing 2 mg/ml lysozyme and 2 mM EDTA, and the suspension was allowed to stand at 0°C for 30 minutes. The suspension was further ultrasonicated and centrifuged to obtain an insoluble fraction as a precipitate.

The insoluble fraction thus obtained was solubilized by the addition of 8M urea, and centrifuged to collect a supernatant. The supernatant was subjected to column chromatography using DEAE Toyopeal (TOSO Co.) (eluate: A = 20 mM Tris-HCl/6M urea (pH 8.0); B = 0.5M NaCℓ/eluate A (pH 8.0); concentration gradient: from A to B with linear gradient/300 minutes; column φ1.6 × 40 cm; flow rate 1 ml/min.) to isolate and purify the desired protein. The eluted fractions were gathered, dialysed and lyophilized. The protein was cleaved with cyanogen bromide to remove TrpE moiety. The cyanogen bromide cleavage was carried out by adding the protein to a 70% formic acid solution to a protein concentration of 1%, adding 100 equivalents of cyanogen bromide thereto, and the resulting reaction mixture was allowed to stand at 37°C for 24 hours. The mixture was dialysed, lyophilized and subjected to reversed phase high performance liquid chromatography (eluate: A = 0.1% trifluoroacetic acid/water, B = 60% acetonitryle/0.1% trifluoroacetic acid/water) to purify and obtain 30 mg of GRP protein. Purity of the protein was homrogeneous as determined by reversed phase column chromatography and SDS-polyacrylamide gel electropholesis. Moreover, it was confirmed that the protein has the amino acid sequence of GRP as determined by a peptide sequencer.

### Example 2. Production of polyclonal antibody

The product GRP(31-98) thus prepared was conjugated with KLH as a carrier protein, and 150 µg of the conjugate was first injected to a rabbit NZW, and further injections of 100 µg after two weeks, 100 µg after 2 weeks, 85 µg after 6 days, 80 µg after 12 days and 60 µg after 12 days were made. After an increase of antibody titer was confirmed, the whole blood was obtained.

### Example 3. Production of monoclonal antibody

GRP(31-98) was conjugated with KLH as carrier protein, and the conjugate was first injected to a Balb/c mouse, and further injections of 50 µg after 3 weeks, 50 µg after three weeks, 50 µg after 4 weeks and 30 µg after 4 weeks were made.

Spleen cells (1 × 10⁸ cells/ml) from the immunized mouse were mixed with previously cultured myeloma cells P3U1 (1 × 10⁷ cells/ml) at a mixing ratio of 10:1 by cell amount, and the mixture was incubated at 37°C for 5 minutes.

Next, to the mixture was added 50% polyethylene glycol 1500 followed by RPMI 1640 medium. After centrifugation, HAT medium containing 20% FCS was added thereto, and after adjusting the cell concentration to 10⁵ cells/ml, the cell suspension was distributed to wells of a 96 well microtiter plate at a volume of 100 µl/well. Next, culturing was carried out at 37°C for 10 to 14 days.

To an ELISA plate coated with 100 ng/ml GRP(31-98) was added 100 µl of the above-obtained culture supernatant and was reacted for 2 hours. After reaction with peroxidase-labeled anti-mouse immunoglobulin G antibody for 1.5 hours, the reaction mixture was developed with a 33',55'-tetramethylbenzidine (TMBZ) solution. Absorbance at 450 nm was measured, and clones providing at least 0.3 of the absorbance was taken as positive to obtain hybridoma that produces a desired monoclonal antibody. The hybridoma cells were intraperioneally inoculated to mice treated with pristan, and the monoclonal antibody produced in the ascites was recovered. Alternatively, the hybridoma cells were cultured in vitro in a medium, and monoclonal antibody was obtained from the culture supernatant.

The monoclonal antibody was purified according to a conventional procedure by ammonium sulfate precipitation, dialysis with a phosphate buffer and purification by a protein A- linked Sepharase column to obtain an IgG fraction.

The affinity of the monoclonal antibody thus prepared to GRP(31-98) antigen was determined as follows. The GRP(31-98) antigen was immobilized to a microliter plate to saturation, and to the plate was added 20 µl of a solution containing 0 to 0.3 µg/ml monoclonal antibody to the GRP(31-98) prepared by dilution of the monoclonal antibody (10 µg/ml), and allowed to react. The antibody bonded to the immobilized GRP(31-98) was measured using an enzyme-labeled anti-mouse IgG.

The amount of the bonded antibody [B] was obtained from the relationship between the values of absorbance measured and amounts of the antibody added, and the amount of free antibody (F) was obtained by subtracting an amount of the bonded antibody from an amount of the antibody added. The amount of the bonded antibody [B] was converted to a molar concentration assuming a molecular weight of the antibody of 150,000 daltons, and to the obtained molar concentrations the values of B/F were Scatchard-platted. An association constant Ka = 3 × 10⁸ ~ 2 × 10¹⁰/M was obtained from the slope of Scatchard plotting.

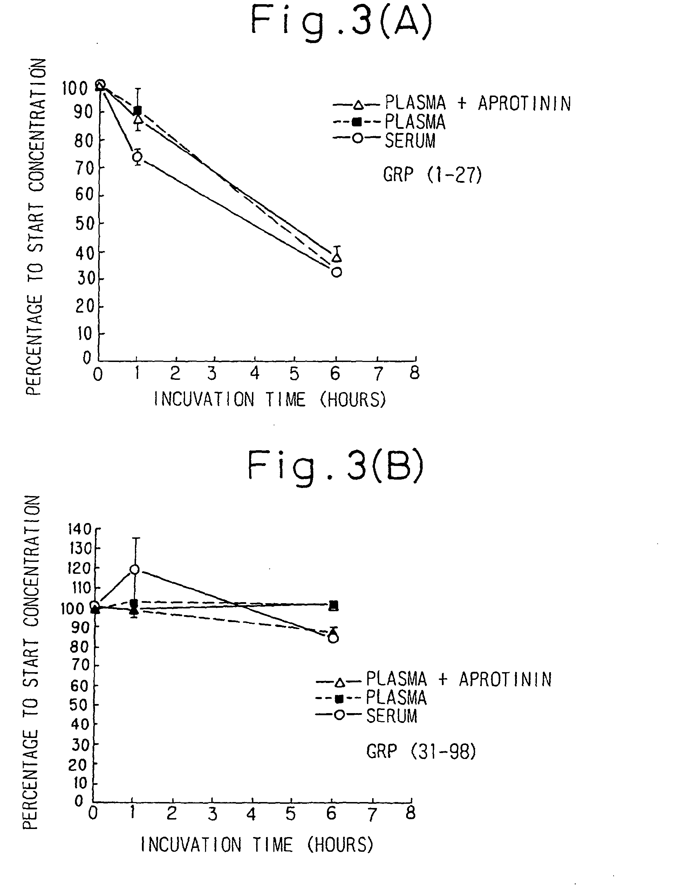

### Example 4. Comparison of stability of GRPs in the blood (Fig. 3)

Stabilities in the blood of GRP(1-27) and GRP(31-98) were compared using antibodies to the above-prepared GRP(31-98) and antibodies to the conventional GRP(1-27).

A small amount of GRP(1-27) or GRP(31-98) was added to a serum, plasma, or aprotinin - containing plasma, and the residual concentration of GRP(1-27) and GRP(31-98) was determined at 0, 1, and 6 hours from the addition, and the percentages of the residual concentration were obtained taking the concentration at the starting point (0 hour) as 100%. As seen from Fig. 3, although the active peptide GRP(1-27) was degraded and disappeared in the presence of serum or plasma, the peptide GRP(31-98) was stable for a long time in the serum or plasma.

### Example 5. Preparation of monoclonal antibody

The GRP(31-98) prepared as described above was conjugated to a carrier protein, thyroglobulin, the conjugate was dissolved in phosphate buffer (pH 7.4)(PBS(-)) to a concentration of 1.0 mg/ml, and the solution was mixed with an equal volume of Freund's complete adjuvant to form a suspension. An amount of the suspension thus prepared containing 0.01 to 0.05 mg of GRP(31-98) was intraperitoneally administered to a BALB/C mouse of 4 to 6 weeks old. After about 12 weeks, the immunized animal was administered with the same concentration of GRP(31-98) solution in PBS(-1) containing about 0.01 to 0.03 mg of GRP(31-98), through a tail veins. After 3 days from the administration, the spleen was aseptically removed from the immunized animal.

Next, the spleen was disrupted to single cells by a mesh, and the cells were washed three-times with RPMI-1640 medium. Mouse myeloma cells p3 × 63 Ag 8 (Nature, 256, 495-497, 1975) in the logarithmic growth phase were cultured for a few days in the presence of 8-azaguanine to completely eliminate reverse mutants, and washed as described above. 1.1 × 10⁷ cells of the mouse myeloma cell line and 1.4 × 10⁸ spleen cells prepared as described above were mixed. After centrifugation at 200 × g for 5 minutes, the supernatant was replaced with 1 ml of RPMI-1640 medium containing 50% PEG 4000 (Merck) warmed at 37°C for cell fusion.

The cells subjected to the cell fusion were centrifuged to remove PEG, and cultured in RPMI-1640 medium containing hypoxanthine, aminopurine and thymidine (abbreviated as HAT hereinafter) as well as 15% fetal calf serum (FCS) for 1 to 2 weeks to allow for hybridoma exclusively to grow. Next, the hybridoma cells were grown in a HAT-free medium for about two weeks, and clones were screened by ELISA as described below to obtain hybridoma producing a monoclonal antibody of the present invention showing a desired reaction specificity.

The ELISA was carried out as follows. GRP(31-98) was dissolved in phosphate buffer (pH 7.4)(PBS) to a concentration of 1 µg/ml, and 50 µl of the solution was distributed to each well of a 96-well microliter plate, and the plate was incubated overnight at 4°C or for one hour at room temperature for adsorption. After the adsorption, the wells were washed three times with 0.05% Tween-20-containing PBS(-) (abbreviated as T-PBS), and 200 µl/well of 1% BSA-containing PBS(-) was distributed and the plate was incubated at room temperature for one hour. The 1% BSA-containing PBS(-) was removed, and 50 µl/well of hybridoma culture supernatant, crude monoclonal antibody or purified monoclonal antibody was added, followed by reaction for one hour at a room temperature. After the reaction, wells were washed three times with T-PBS, and 50 µl/well of an enzyme-labeled mouse IgG+M antibody (Jackson) diluted 5000-fold with PBS(-) containing 1% BSA, 1% polyvinylpyrrolidone and 0.05% Tween-20 was added, and reaction was carried out at a room temperature for 30 minutes. Non-reacted antibody was eliminated by 4 washings with T-PBS, and 50 µl/well of orthophenylenediamine (OPD) solution (Wako Pure Chemicals) was added for reaction. After 20 minutes of incubation at room temperature, the reaction was terminated with 2N sulfuric acid, and absorbance at 492 nm was measured.

The hybridoma thus obtained were designated as proGRP-1E2, proGRP-2B10, proGRP-20D2, pro-GRP-3H1, proGRP-3D6, proGRP-3G2 and proGRP-4C9, and among them the hybridoma proCRP-2B10 and proGPP-3G2 were deposited with the Formentation Research Institute, Agency of Industrial Science and Technology (FRI) under the Budapest treaty on December 9, 1992 as FERM BP-4110 and FERR BP-4109 respectively. Moreover, the hybridoma proGRP-20D2 was designated as "anti-proGRP monoclonal antibody-producing hybridoma (20D2) and was deposited with the FRI under the Budapest Treaty on February 10, 1993 as FERM BP-4184.

According to double immunodiffusion using rabbit anti-mouse Ig isotype antibodies (Zymed), the isotypes of the monoclonal antibodies produced by the above-mentioned hybridoma were determined as follows. The monoclonal antibodies GRP-1E2, GRP-2B10 and GRP-3G2 produced by the hybridoma proGRP-1E2, proGRP-2B10 and proGRP-3G2 respectively were IgG1; the monoclonal antibodies GRP-20D2 and GRP-3D6 produced by the hybridoma proGRP-20D2 and proGRP-3D6 was IgG2; and the monoclonal antibody GRP-3H1 produced by the hybridoma proGRP-3H1 was IgM.

Note that, "proGRP" means precusor of GRP.

### Example 6. ELISA using monoclonal antibodies

The hybridoma proGRP-2B10, proGRP-1E2, proGRP-20D2, proGRP-3D6, proGRP-3H1 and proGRP-409 were intraperitoneally inoculated in mice, and from the ascites, corresponding monoclonal antibodies GRP-2B10, GRP-1E2, GRP-20D2, GRP-3D6, GRP-3H1 and GRP-4C9 with at least 90% purity were obtained using a Protein-A column, gel-filtration column or Protein-G column.

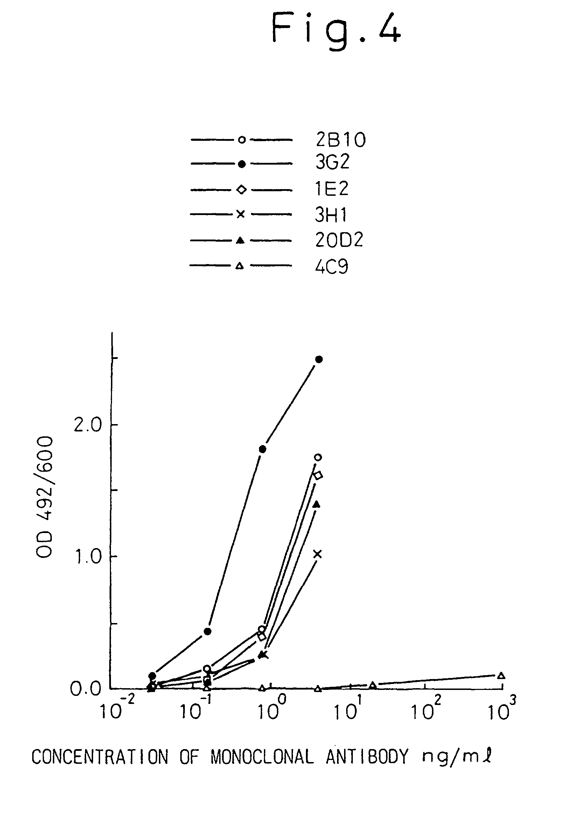

ELISA was carried out as follows. GRP(31-98) was dissolved in a phosphate buffer (pH 7.4)(PBS) to a concentration of 1 µg/ml, and 50 µl of the solution was distributed to each well of a 96-well microplate, and the plate was incubated overnight at 4°C or for one hour at room temperature for adsorption. After the adsorption, the wells were washed three times with 0.05% Tween-20-containing PBS(-) (abbreviated as T-PBS), and 200 µl/well of 1% BSA-containing PBS(-) was distributed and the plate was incubated at a room temperature for one hour. The 1% BSA-containing PBS(-) was removed, and 50 µl/well of hybridoma culture supernatant, crude monoclonal antibody or purified monoclonal antibody was added, followed by reaction for one hour at room temperature. After the reaction, the wells were washed three times with T-PBS, and 50 µl/well of an enzyme-labeled mouse IgG+M antibody (Jackson) diluted 5000-fold with PBS(-) containing 1% BSA, 1% polyvinylpyrrolidone and 0.05% Tween-20 was added, and reaction was carried out at room temperature for 30 minutes. Non-reacted antibody was eliminated by 4 washings with T-PBS, and 50 µl/well of orthophenylenediamine (OPD) solution (Wako Pure Chemicals) was added for reaction. After 20 minutes of incubation at room temperature, the reaction was terminated with 2N sulfuric acid, and absorbance at 492 nm was measured. The result is shown in Fig. 4. As seen from Fig. 4, monoclonal antibody most highly reactive with proGRP (GRP precursor) is GRP-3G2, followed by GRP-2B10, GRP-1E2, GRP-20D2, GRP-3H1 and GRP-4C9 in this order.

### Example 7. Production of polyclonal antibody

The polypeptide GRP(31-98) prepared as described above was conjugated to a carrier protein, thyroglobulin, the conjugate was dissolved in a phosphate buffer (pH 7.4) (PBS(-)) to a concentration of 1.0 mg/ml, and the solution was mixed with an equal volume of Freund's complete adjuvant to form a suspension. An amount of the suspension thus obtained containing 0.1 mg of GRP(31-98) was subcutaneously administered to 1.5 - 2.0 kg weight rabbits Kbl:JW, 8 weeks old, followed by 6 repeated administrations of 0.07 mg of GRP(31-98) at ten day intervals. The blood from rabbits which provided a high titer was obtained to prepare polyclonal antibody.

### Example 8. Identification of antigenicity in serum and urine

To identify fragment of ProGRP immunogenic in serum samples and urea samples, following experiment was carried out. Various peptides, i.e., GRP(31-98) (SEQ ID NO: 1), GRP(31-52) (SEQ ID NO: 10), GRP(44-62) (SEQ ID NO: 11), and GRP(70-90) (SEQ ID NO: 12) were covelently immobilized to wells of a microplate, and to the wells were added 50 µl each of 9 serum samples, one urine sample and standard peptide (GRP(31-98)) solutions. Immediately after the addition of the sample or standard, 150 µl of anti-GRP(31-98) antiserum diluted with a reaction buffer (1% BSA, 0.05% Tween 20, 0.15M NaCl, 0.1M phosphate pH 7.0) was added to each well, and the plate was incubated at 4°C overnight. During this incubation, the peptide immobilized on the plate and the peptide contained in the sample or standard compete against the anti-GRP(31-98) antiserum, and as a result, if the peptide contained in the sample is antigenic the antiserum preferentially reacts with the peptide in the sample but not with the peptide immobilized on the plate, and therefore the amount of the antiserum which binds to the peptide immobilized on the plate is small.

Next, the plate was washed 5 times with a washing solution, a peroxidase-labeled anti-rabbit 1gG was added to each well, and an incubation was carried out at room temperature for one hour. After washing the plate 5 times with a washing solution, an orthophenylenediamine substrate was added to each well, an incubation was carried out for one hour and the reaction was terminated by adding sulfuric acid.

The absorbance at 492 nm was measured, and a standard curve for each peptide was prepared. After that the ProGRP antigenic activity in each sample was determined using the standard curve.

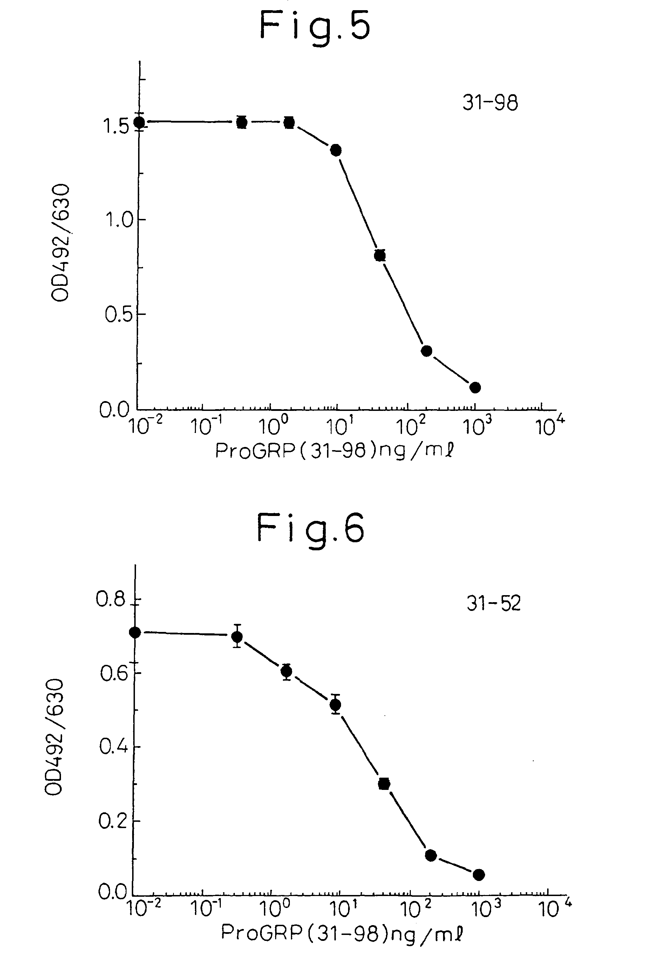

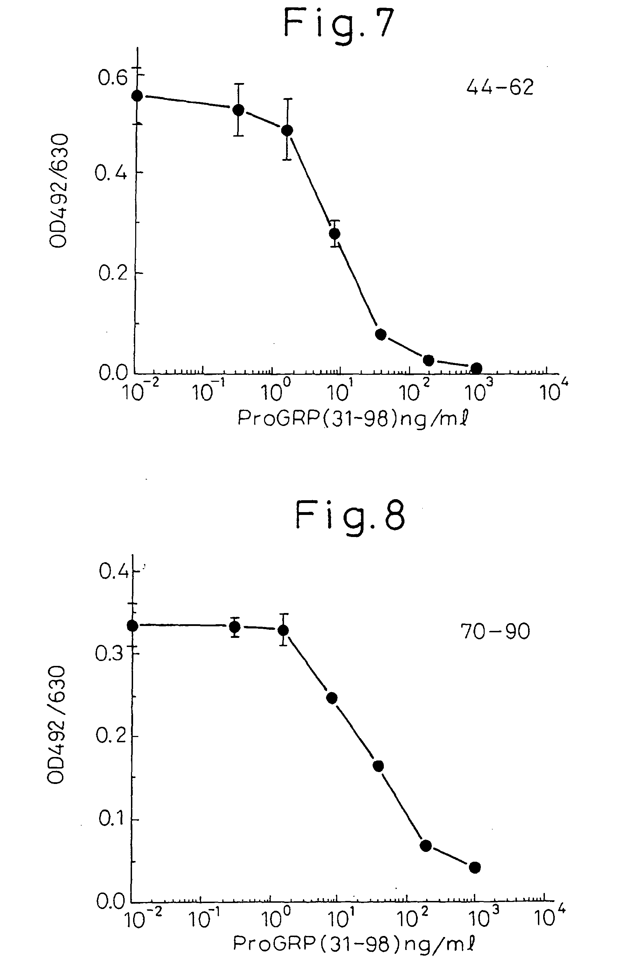

A standard curve for each peptide is shown in Figs. 5 to 8. The results of measurement of the samples are shown in Table 2.

**Table 2**

| Antigenicity of ProGRP fragment in serum or urine | | | | | | | |
|---|---|---|---|---|---|---|---|
| Sample No. | 31-98 | 31-52 | | 44-62 | | 70-90 | |
| | ng/ml | ng/ml Z of(31-98) | | ng/ml % of (31-98) | | ng/ml % of (31-98) | |
| Serum 1 | 5.4 | 8.4 | 155.6 | 3.6 | 66.7 | 13.5 | 250.0 |
| Serum 2 | 47.0 | 36.0 | 76.6 | 40.0 | 85.1 | 47.5 | 101.0 |
| Serum 3 | 10.0 | 17.5 | 175.0 | 5.8 | 58.0 | 21.8 | 218.0 |
| Serum 4 | 11.4 | 14.0 | 122.8 | 8.6 | 75.4 | 19.5 | 171.1 |
| Serum 5 | 23.5 | 22.0 | 93.6 | 24.5 | 104.3 | 44.0 | 187.2 |
| Serum 6 | 138.0 | 54.0 | 39.1 | 90.0 | 65.2 | 108.0 | 78.3 |
| Serum 7 | 295.0 | 30.0 | 10.2 | 140.0 | 47.5 | 198.0 | 67.1 |
| Serum 8 | 225.0 | 22.5 | 10.0 | 230.0 | 102.2 | 265.0 | 117.8 |
| Serum 9 | 12.0 | 28.0 | 233.3 | 12.0 | 100.0 | 5.7 | 47.5 |
| mean | | | 101.8 | | 78.3 | | 137.6 |
| urine 1 | 75.0 | 3.8 | 5.1 | 1.8 | 2.4 | 140.0 | 186.7 |
| total mean | | | 93.0 | | 71.4 | | 142.0 |

For serum 2, the antigenicity of the three peptides are almost same and are not substantially different from that of GRP(31-98). For sera 4 and 5, the antigenicity of GRP(31-52) and GRP(44-62) was almost same as that of GRP(31-98), while to antigenicity of GRP(70-90) is two times that of GRP(31-98). For sera 6, 7 and 8, the antigenicity of GRP(31-52) is low, the antigenicity of GRP(44-62) and GRP(70-90) are relatively maintained. For serum 9, the antigenicity of GRP(70-90) is maintained in comparison with that of GRP(31-62), and is about 138% of GRP(31-98).

For the urine sample, the antigenicity of GRP(70-90) is very high, while the antigenicity of GRP(31-52) and GRP(44-62) are absent. Especially, for the sera 7 and 8, and the urine, the antigenicity of GRP(31-52) is about 10% or less of that of GRP(31-98), revealing that an antibody against a portion other than 31-52 of ProGRP is preferable for detection of ProGRP immune activity.

From the above-mentioned results it is considered that after secrection into the blood, the ProGRP is degraded and modified during the circulation, and a region containing the 70-90 portion is stably maintained in comparison with other peptides, and excreted from the kidney, while the N-terminal side of ProGRP is degraded and is not excreted from the kidney. Accordingly, it was found that to assay ProGRP fragments in a serum or urine sample, an antibody or receptor capable of binding to the region of 63-98 including the 70-90 portion of the ProGRP is important.

### Example 9. Analysis of epitope of monoclonal antibodies

Various peptides, i.e., GRP(31-52), GRP(44-62), GRP(70-90) and GRP(31-98) were immobilized on wells of a microliter plate. For a control, no peptide was immobilized. To the plate, different amounts of various monoclonal antibodies to be tested were added to allow the reaction of each of the peptides and each of the monoclonal antibodies. After washing the plate, a peroxidase-conjugated anti-mouse Ig(G + M) was added to each well and the plate was incubated. After washing, an enzymatic reaction was allowed using as a substrate orthophenylenediamine at a room temperature for 30 minutes, and the reaction was terminated by adding sulfuric acid. Immediately after the addition of sulfuric acid, the absorbance at 492 nm was measured. The results are shown in Table 3A. If monoclonal antibody reacts with a peptide, a higher signal is provided, while if the monoclonal antibody does not react with a peptide, a lower signal is provided. As can be seen from Table 3A, the monoclonal antibodies 2B10, 1E2, 20D2, 3G2 and 3H1 recognize with the peptide GRP(70-90); the monoclonal antibody 3D6 recognizes the peptide GRP(44-62); and the monoclonal antibody 4C9 recognizes the peptide GRP(31-52).

To confirm the above-mentioned result, the following peptide inhibition test was carried out. Namely, different amounts of various monoclonal antibodies to be tested and 5 µg/ml of the various peptides were combined and incubated at a room temperature for 2 hours. The reaction mixture was added to a microtiter plate on which the peptide GRP(31-98) had been immobilized, and the microliter plate was incubated at a room temperature for one hour so as to allow a competitive reaction of the monoclonal antibody with the peptide in the solution and the peptide GRP(31-98) immobilized on the plate. After washing the plate, peroxidase-conjugated anti-mouse Ig(G + M) was added thereon, and the plate was incubated to allow a reaction of the labeled Ig(G + M) with the monoclonal antibody which bound to the immobilized peptide GRP(31-98).

After washing the plate, orthophenylenediamine as a substrate was added to the plate, and an enzymatic reaction was carried out at room temperature and terminated with addition of sulfuric acid. Immediately after the termination of the reaction, the absorbance at 492 nm was measured. For each monoclonal antibody, a result obtained from wells to which no peptide had been immobilized was taken as a control. If a monoclonal antibody reacts with a peptide in the solution a lower signal is provided; while if a monoclonal antibody does not react with a peptide in the solution, the monoclonal antibody reacts with the immobilized peptide GRP(31-98) resulting in a higher signal.

As can be seen from Table 4B, the reaction of the monoclonal antibodies 2B10, 1E2, 20D2, 3G2 and 3H1 with the immobilized peptide GRP(31-98) was inhibited with the peptide GRP(70-90), revealing that these monoclonal antibodies recognize an epitope present in the GRP(70-90). The reaction of the monoclonal antibody 4C9 with the immobilized peptide GRP(31-98) was significantly inhibited only by the peptide GRP(31-52) but not by the peptide GRP(44-62), revealing a high possibility that the monoclonal antibody 4C9 recognizes an N-terminal site of the peptide GRP(31-52).

**Table 3**

| Identification of epitope for monoclonal antibodies A. Peptide direct assay | | | | | | | |
|---|---|---|---|---|---|---|---|
| Peptides | Monoclonal antibodies | | | | | | |
| | 2B10 | 3G2 | 1E2 | 3H1 | 3D6 | 20D2 | 4C9 |
| GRP(31-52) | 0.002 | 0.001 | 0.017 | 0.019 | 0.002 | 0.000 | 0.161 |
| GRP(44-62) | 0.004 | 0.001 | 0.015 | 0.032 | 0.168 | 0.000 | 0.004 |
| GRP(70-90) | 1.059 | 0.741 | 0.803 | 1.086 | 0.003 | 1.202 | 0.004 |
| GRP(31-98) | 1.058 | 2.075 | 2.726 | 0.949 | 0.085 | 1.042 | 1.409 |
| No | 0.000 | 0.002 | 0.006 | 0.000 | 0.003 | 0.000 | 0.003 |

| B. Peptide Competitive assay (% of control) | | | | | | | |
|---|---|---|---|---|---|---|---|
| Comparative Peptides | | Monoclonal antibodies | | | | | |
| | 2B10 | 3G2 | 1E2 | 3H1 | 20D2 | 4C9 | |
| GRP(31-52) | 102.1 | 112.2 | 82.8 | 81.5 | 111.0 | 1.8 | |
| GRP(44-62) | 104.6 | 102.6 | 105.0 | 106.7 | 99.3 | 100.3 | |
| GRP(70-90) | 0.9 | 0.3 | 1.5 | 3.3 | 18.8 | 106.9 | |
| GRP(31-98) | 19.5 | 10.1 | 11.8 | 12.0 | 28.2 | 76.0 | |

From Table 3, it was confirmed that the monoclonal antibodies 2B10, 1E2, 20D2, 3G2 and 3H1 recognize an epitope in the peptide GRP(70-90)

### Example 10. Detection (1) of ProGRP in urine or serum sample

A mixture of the monoclonal antibodies 2B10 and 3G2 was adsorbed on each well of a microliter plate, and after adding 100 µl/well of a reaction buffer (1% BSA, 0.05% Tween 20, 0.5M NaCl, 0.1M phosphate pH 7.0), 50 µl each of serum sample or a standard solution containing a known concentration of the peptide GPR(31-98) was added to each well. The plate was incubated at 37°C for 60 minutes. Each well was washed 5 times with about 350 µl of a washing solution (0.01M phosphate, 0.15M NaCl, 0.05% Tween 20 pH 7.3). Then 100 µl of a solution of a peroxidase-labeled anti ProGRP rabbit IgG was added to each well, and the plate was incubated at room temperature (20 to 30°C) for 30 minutes. Each well was washed 5 times with about 350 µl of a washing solution and 100 µm of a substrate solution of orthophenylenediamine was added to each well. The plate was allowed to stand in the dark at room temperature for 30 minutes, and 100 µl of a reaction termination reagent was added.

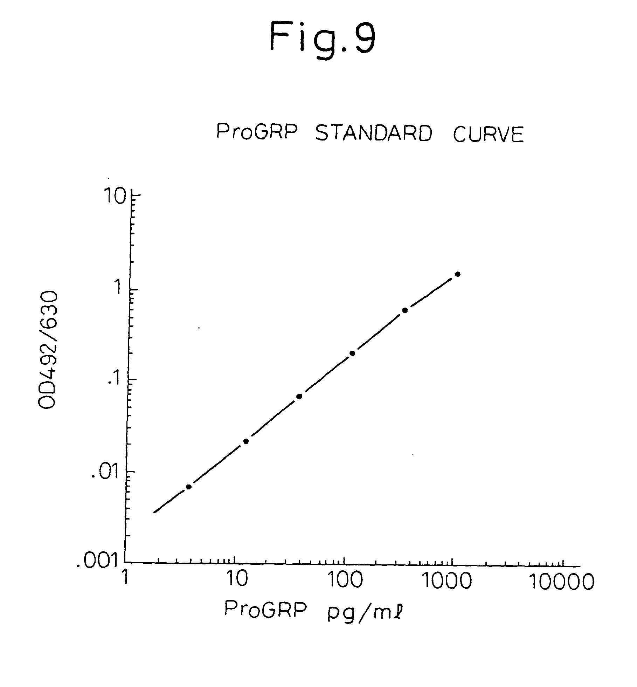

The absorbance at 492 nm, or in case of two wavelengths, at 492 nm and 600 to 700 nm (as reference) was measured taking a blank well as a control, using a microplate reader. A standard curve obtained from the standards is shown in Fig. 9. The results are shown in Table 4.

**Table 4**

| | Number of samples (n) | Number of positive samples | Ratio of positive sample (%) | Concentration of peptide (ng/L) mean±SD |
|---|---|---|---|---|
| Sera from healthy subject | 247 | 1 | 0.4 | 12.6 ± 6.9 |
| Sera form patients with benign lung diseases | 20 | 0 | 0.0 | 15.2 ± 6.7 |
| Sera from patients with lung cancer non-SCLC | 20 | 1 | 5.0 | 16.6 ± 12.0 |
| SCLC Limited diseases | 12 | 8 | 66.7 | 861.9 ± 1203.3 |
| Extend diseases | 13 | 10 | 76.9 | 1645.0 ± 1490.6 |

The urea samples obtained from the same subjects or patients were analyzed by the above-mentioned procedure. The results are shown in Table 5.

**Table 5**

| Urea sample from | ProGRP (pg/ml) | |
|---|---|---|
| Healthy subject | 1 | < 3 |
| | 2 | < 3 |
| | 3 | < 3 |
| Patient with small cell lung | 1 | < 3 |
| cancer | 2 | <3 |
| | 3 | < 3 |
| | 4 | 88.8 |

As can be seen from Tables 4 and 5, when various serum samples and urea samples were measured, in the case wherein two monoclonal antibodies is used as a first antibody and a polyclonal antibody is used as a second antibody, serum samples from patients with small cell lung cancer provided higher values and serum samples from healthy subjects provided lower values. On the other hand, in the same assay procedure, a urea sample could not be measured.

### Example 11. Detection (2) of ProGRP in urine or serum sample

Monoclonal antibody 3G2 was absorbed on wells of a microliter plate, and after adding 100 µl/ well of a reaction medium (1% BSA, 0.05% Tween 20, 0.15M NaCl, 0.1M phosphate buffer pH 7.0), 100 µl each of a urea sample or a standard solution containing a known concentration of the peptide GPR(31-98) was added to each well. Incubation was carried out at room temperature for 2 hours. Each well was washed 5 times with about 350 µl of a washing solution (0.01M phosphate, 0.15M NaCl, 0.05% Tween 20, pH 7.3). 200 µl of a solution of peroxidase-labeled monoclonal antibody 2B10 was added to each well, and reacted at room temperature (20°C to 30°C) for 60 minutes. Each well was washed 5 times with about 350 µl of a washing solution. 200 µl of a substrate solution of orthophenylenediamine was added to each well, and after allowing it to stand for 60 minutes at room temperature in the dark, 50 µl of a reaction termination reagent (2N sulfuric acid) was added to each well. The absorbance at 492 nm, or in case of two wavelengths, at 492 nm and 600 to 700 nm (as reference) was measured taking a blank well as a control, using a microplate reader. Using a standard curve obtained from the standards of GRP(31-98), concentration of ProGRP(pg/ml) for each sample was obtained. The results are shown in Table 6 and 7.

**Table 6**

| Urea sample from | | ProGRP (pg/ml) |
|---|---|---|
| Healthy subject | 1 | 6.5 |
| | 2 | 5.1 |
| | 3 | 5.2 |
| Patient with small | 1 | 282 |
| cell lung cancer | 2 | 171 |
| | 3 | 283 |
| | 4 | 17233 |

**Table 7**

| Serum samples from | | ProGRP (pg/ml) |
|---|---|---|
| Healthy subject | 1 | 20.2 |
| | 2 | 35.2 |
| | 3 | 15.2 |
| | 4 | 24.0 |
| | 5 | 22.3 |
| | 6 | 16.5 |
| | 7 | 38.5 |
| | 8 | 21.6 |
| Patient with small | 1 | 400.9 |
| cell lung cancer | 2 | 415.5 |
| | 3 | 550.9 |
| | 4 | 186.4 |
| | 5 | 1874.2 |

As can be seen from Table 6, ProGRP immune activity in urea sample can be measured by using monoclonal antibodies recognizing the peptide GRP(70-90) both as the first and second antibodies. In addition it was confirmed that higher ProGRP immune activity is present in a urea sample from a patient having small cell lung cancer than in a urea sample from a healthy subject. Using this ELISA, 8 sera from healthy subjects and 5 sera from patients with small cell lung cancer were tested, and the results are shown in Table 7, a result similar to that in Table 4 in Example 10 was obtained.

### Example 12. Gel permeation chromatography analysis of ProGRP antigenicity in serum and urine

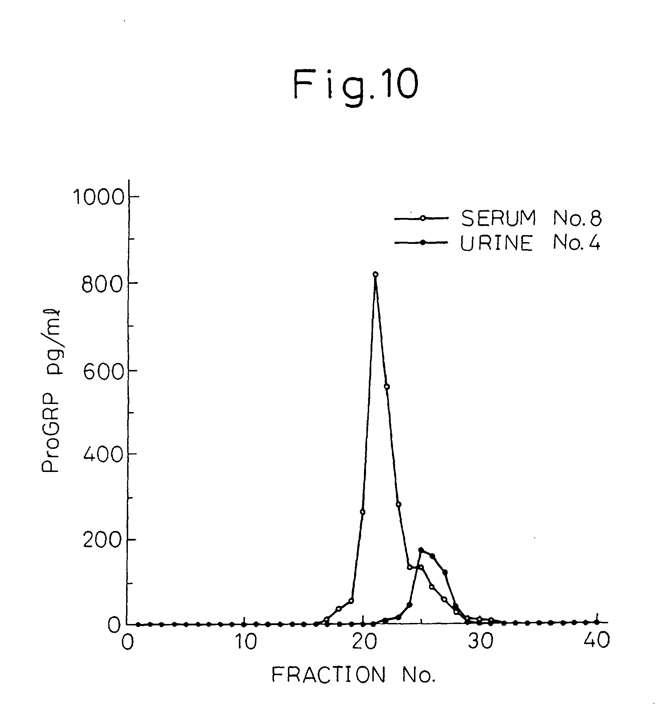

100 µm of a serum sample or a urine sample was applied to a Superde X 30 pg (1 × 50) column swollen with a carbonate buffer (pH 8.0), and the immune activity of elute fractions was measured by the assay method described in Example 11. The results are shown in Fig. 10. From the results, it was confirmed that an immune activity of the serum sample has a main peak at the fraction Nos. 21 to 22 and a minor peak at a low molecular side of the main peak, which minor peak was supposed to represent a degradation product the peptide corresponding to the main peak. For the urine sample, no peak was observed at fraction Nos. 21 to 22, but only a peak at the fraction Nos. 25 to 26, corresponding to the peak supposed to be a degradation product in the serum sample, was observed. From this result, it was supposed that there are mainly protein having a molecular weight of 10,000 to 12,000 in the serum, and during circulation in the body, the proteins are degraded, and the degradation product is rapidly excreted into urine, and as a result, urine contains peptides having a molecular weight of 2,500 to 7,000, including mainly the peptide GRP(70-90).

## Claims

1. A method for detecting or assaying the fragments of GRP precursor (ProGRP) in a urine sample by using monoclonal antibodies binding to the 70-90 portion of the ProGRP as a first antibody and a second antibody.

2. A method according to claim 1 for diagnosis of small cell lunger cancer.

## Patentansprüche

1. Verfahren zum Nachweisen oder Untersuchen der Fragmente des GRP-Vorläufers (ProGRP) in einer Urinprobe durch Verwenden von monoklonalen Antikörpern, die an den Bereich 70-90 des ProGRP binden, als einen ersten Antikörper und einen zweiten Antikörper.

2. Verfahren nach Ansprüch 1 zur Diagnose von kleinzelligem Lungenkrebs.

## Revendications

1. Procédé pour détecter et doser les fragments du précurseur du GRP (ProGRP) dans un échantillon d'urine en utilisant des anticorps monoclonaux se liant à la partie 70-90 du ProGRP en tant qu'anticorps primaire, et un anticorps secondaire.

2. Un procédé selon la revendication 1 pour le diagnostique du cancer du poumon à petites cellules.
